Europäisches Patentamt

European Patent Office

Office européen des brevets

(19)

(11) Numéro de publication: **0 317 394 B1**

(12)

# FASCICULE DE BREVET EUROPEEN

(45) Date de publication de fascicule du brevet: **26.01.94**

(21) Numéro de dépôt: **88402809.3**

(22) Date de dépôt: **08.11.88**

(51) Int. Cl.[5]: **C07C 51/56**, C07C 57/13,
C07C 63/16, C07C 55/10,
C07C 55/12, C07C 61/22,
C07C 61/09, C07C 63/307,
C07C 63/313

(54) **Procédé de préparation d'anhydrides d'acide.**

(30) Priorité: **20.11.87 FR 8716065**

(43) Date de publication de la demande:
**24.05.89 Bulletin 89/21**

(45) Mention de la délivrance du brevet:
**26.01.94 Bulletin 94/04**

(84) Etats contractants désignés:
**AT BE CH DE ES FR GB GR IT LI LU NL SE**

(56) Documents cités:
**DE-A- 1 094 389
DE-A- 1 173 442
FR-A- 1 315 577
GB-A- 801 722**

**J. CHEM. RESEARCH (S), 1985, pages
356-357; R.W. McCABE et al.: "Clay- and Zeo-
lite-catalysed cyclic anhydride formation"**

**ERDÖL UND KOHLE, vol. 12, no. 5, mai 1959,
pages 335-339, Hamburg, DE; W. FRANZ et
al.: "Katalysatoren auf der Basis säureaktivierter Bentonite"**

(73) Titulaire: **RHONE-POULENC CHIMIE
25, quai Paul Doumer
F-92408 Courbevoie Cédex(FR)**

(72) Inventeur: **Alas, Michel
12, rue Eloi Ricard
F-79500 Melle(FR)**
Inventeur: **Gubelmann, Michel
39, boulevard des Belges
F-69006 Lyon(FR)**
Inventeur: **Popa, Jean-Michel
2, rue Roger Breton
F-93700 Drancy(FR)**

(74) Mandataire: **Le Pennec, Magali et al
RHONE-POULENC INTERSERVICES
Service Brevets Chimie
25, Ouai Paul Doumer
F-92408 Courbevoie Cédex (FR)**

**Description**

La présente invention concerne un procédé de préparation d'anhydrides d'acide. Elle concerne plus particulièrement un procédé qui consiste à préparer sélectivement l'anhydride itaconique à partir de l'acide itaconique.

Il est connu dans l'art antérieur selon les procédés classiques de transanhydridation de préparer l'anhydride itaconique par échange entre l'acide itaconique et l'anhydride acétique. Ce procédé présente l'inconvénient d'utiliser une matière première onéreuse qu'est l'anhydride acétique avec formation d'un produit secondaire qu'il faudra éliminer : l'acide acétique. La réaction est en plus limitée quant à la température utilisée par crainte de voir une réaction de polymérisation se produire. Une température inférieure à 75°C est impérative. Les rendements obtenus par ce procédé ne dépassent pas 90 % anhydride itaconique. Aussi l'industrie cherche depuis longtemps à effectuer l'anhydridation de l'acide itaconique directement à partir de l'acide sans être obligé d'utiliser un autre anhydride tel que l'anhydride acétique.

Il est aussi connu d'après l'article paru dans J. Chem. Research (S) 1985, 356-357, de préparer l'anhydride itaconique par passage de l'acide itaconique sur une argile commerciale du type TONSIL 13 de Süd-Chemie (Münich) traitée avec un sel d'aluminium. Cette technique utilise des argiles échangées avec un sel d'aluminium. Elle présente d'une part l'inconvénient de nécessiter un traitement de l'argile naturelle assez élaboré qui grève le coût d'obtention de l'anhydride. Le catalyseur évolue d'autre part dans le temps sous une forme moins active.

Il a maintenant été découvert un nouveau procédé d'obtention des anhydrides d'acide qui permet d'éviter les inconvénients des procédés de l'art antérieur.

Ce procédé consiste à mettre en présence l'acide avec une argile ayant subi un ou plusieurs traitements acides.

Les argiles utilisables dans le procédé de la présente invention sont choisis de préférence parmi les argiles naturelles présentant une structure dite "TOT" ou tetraèdre-octaèdre-tétraèdre.

Ces argiles sont divisées en 3 classes :
- les smectites,
- les vermiculites,
- les micas.

Les argiles "TOT" se présentent sous forme de feuillets élémentaires comprenant deux couches de tétraèdres d'atomes d'oxygène dans lesquels sont inclus les atomes de silicium séparées par une couche d'octaèdres d'atomes d'oxygène dans lesquels est inclus le métal M de type $(MO_4OH_2)$ où M est un cation di ou trivalent.

Lorsque tous les tétraèdres sont occupés par des éléments $Si^{IV}$ la neutralité électrique du feuillet peut être assurée de deux façons, selon la charge du cation octaédrique :
- s'il est divalent $(Mg^{2+}, Fe^{2+}, ...)$, toutes les cavités octaédriques sont occupées. Le feuillet est alors dit trioctaédrique ;
- s'il est trivalent $(Al^{3+}, Fe^{3+}, ...)$, deux cavités octaédriques sur trois sont occupées et le feuillet est dit dioctaédrique.

Mais de nombreuses substitutions sont possibles, aussi bien dans la couche tétraédrique que dans la couche octaédrique. Celles-ci peuvent entraîner un déficit de charge du feuillet et la neutralité du cristal est alors réalisée par insertion de cations compensateurs entre les feuillets.

On préfère parmi les argiles "TOT" précédemment définies utiliser les smectites.

Les smectites sont classifiées selon la nature du métal M (aluminium, magnésium, fer, lithium) et la nature du cation compensateur (sodium, potassium, calcium).

On peut ainsi citer parmi la classe des smectites :
- les montmorillonites de formule générale

$$Si_4(Al_{2-x}\text{-}Mg_x)O_{10}(OH)_2, M^+{}_x$$

- les beidellites de formule $(Si_{4-x}Al_x)Al_2O_{10}(OH)_2, M^+{}_x$
- les nontronites de formule $(Si_{4-x}Al_x)Fe_2O_{10}(OH)_2, M^+{}_x$
- les hectorites de formule $Si_4(Mg_{3-x}\text{-}Li_x)O_{10}(OH)_2, M^+{}_x$
- les stévensites de formule $Si_4(Mg_{3-x})O_{10}(OH)_2, M^+{}_{2x}$
- les saponites de formule $(Si_{4-x}Al_x)Mg_3O_{10}(OH)_2, M^+{}_x$.
- les fluorohectorites de formule $Si_4(Al_{2-x}\text{-}Li_x)O_{10}(F,OH)_2, M^+{}_x$

- les sauconites de formule $(Si_{4-x}Al_x)(Mg_{3-x}Zn_x)O_{10}(OH)_2,M^+_x$

Parmi les smectites on préfère encore tout particulièrement dans le cadre de la présente invention utiliser les montmorillonites. On peut en outre utiliser les argiles commerciales déjà acides telles que notamment les argiles suivantes :

. KSF vendue par Süd-Chemie (Münich),

. K 10 vendue par Süd-Chemie.

L'argile KSF a une surface de 20 à 40 $m^2$/g et une densité de 800 à 850 g/l.

L'argile K 10 a une surface de 220 à 270 $m^2$/g et une densité de 300 à 370 g/l.

Les argiles subissent un traitement acide selon un premier procédé de l'invention par une solution aqueuse d'un acide. La concentration de la solution aqueuse en acide est variable, cependant elle ne devra pas avoir de préférence un pH inférieur à 2 de façon à ne pas détruire l'argile et elle devra contenir une quantité d'ion $H^+$ exprimé en milliéquivalents correspondant à au moins la capacité d'échange de l'argile. On définit la capacité d'échange d'une argile comme le nombre de cations, exprimé en milliéquivalents qui peuvent être échanger sur 100 g d'échantillon.

Cette capacité d'échange (ou charge par demi-maille) varie pour les smectites entre 0,2 et 0,6 et pour les vermiculites entre 0,6 et 0,9. Il est préférable donc dans le cadre de la présente invention d'utiliser une solution d'acide contenant autant d'équivalents $H^+$ qu'il y aura de cations à échanger dans l'argile donc contenant au moins 0,2 à 0,6 $H^+$ par demi maille soit au moins 50 à 150 milliéquivalents acide pour 100 g d'argile.

Parmi les acides utilisés pour acidifier l'acide, on peut citer notamment l'acide chlorhydrique, l'acide sulfurique, l'acide nitrique, l'acide perchlorique et l'acide phosphorique. Les acides organiques pourraient aussi être utilisés notamment l'acide trifluorométhanesulfonique mais ne présentent aucun avantage par rapport aux acides minéraux et présentent surtout l'inconvénient d'être plus onéreux.

Selon un deuxième procédé de traitement acide, l'argile peut être traitée par un sel d'ammonium suivi d'une calcination à basse température de façon à éliminer l'ammoniac en ne laissant subsister que le proton $H^+$. La calcination est effectuée à une température inférieure ou égale à 500°C et de préférence inférieure ou égale à 400°C.

On peut éventuellement, après l'un ou l'autre des procédés de traitement acide, traiter l'argile avec un alcool tel que le méthanol, l'isopropanol ou avec les cétones telle que l'acétone puis la sécher.

L'acide devant subir l'anhydridation est ensuite mis en contact avec l'argile précédemment traitée dans un réacteur en présence d'un solvant choisi parmi les solvants organiques aromatiques éventuellement halogénés notamment le toluène, le xylène, le chlorobenzène et les solvants aliphatiques chlorés.

Pour une meilleure mise en oeuvre de l'invention, on préfère utiliser une quantité pondérale d'acide devant subir l'anhydridation calculée par rapport à une quantité d'argile comprise entre 1 et 20. L'acide peut être introduit de façon continue ou séquentielle. Dans la mesure où le procédé peut être exploité de façon continue, la quantité d'acide calculée par rapport à l'argile peut être nettement supérieure.Une quantité pondérale de solvant calculée par rapport à l'acide comprise entre 20 et 150 est aussi préférée.

Parmi les acides devant subir l'anhydridation, on peut citer :

- les acides aliphatiques dicarboxyliques,
- les acides cycloaliphatiques dicarboxyliques,
- les acides aromatiques polycarboxyliques.

Parmi les acides aliphatiques dicarboxyliques, on peut utiliser les acides saturés ou insaturés, linéaires ou ramifiés contenant de préférence 4 à 8 atomes de carbone. On préfère utiliser les acides aliphatiques dont la chaîne principale est saturée et contient 4 atomes de carbone. On peut citer parmi cette classe l'acide succinique, l'acide itaconique, l'acide maléique, l'acide glutarique. On préfère utiliser l'acide itaconique.

Parmi les acides cycloaliphatiques dicarboxyliques, on peut utiliser les acides saturés ou insaturés partiellement. On préfère néanmoins utiliser les acides saturés. On peut citer dans cette classe l'acide cyclohexenedicarboxylique, l'acide cyclohexanedicarboxylique.

Parmi les acides aromatiques polycarboxyliques, on préfère utiliser les acides aromatiques dicarboxyliques. On peut citer dans cette classe l'acide phtalique, l'acide trimellitique, l'acide trimésique, l'acide pyromellitique, l'acide prehnitique. Les acides substitués par des groupements divers n'influant pas sur la cyclisation de la molécule font aussi partie de l'invention.

Lorsque l'on utilise l'acide itaconique, l'anhydride obtenu présente une sélectivité supérieure à 90 % et une sélectivité en anhydride citraconique inférieure à 5 %.

La réaction entre l'argile et l'acide est réalisée à une température comprise entre 80 et 200°C et de préférence entre 100° et 150°C pour les acides dicarboxyliques. Elle est réalisée de préférence à la pression atmosphérique.

Le produit obtenu par la présente invention c'est-à-dire l'anhydride est exempt de sels métalliques et notamment d'aluminium par comparaison avec l'anhydride itaconique obtenu par le procédé décrit dans la publication parue dans le J.Chem.Research où il existe un risque de voir apparaître des contaminations par un sel d'aluminium. Cette pureté est souvent indispensable car ces anhydrides sont des intermédiaires importants pour l'industrie pharmaceutique et phytosanitaire (brevets US 4 487 777, FR 2 466 450, FR 2 480 600). Ils peuvent aussi être utilisés dans l'industrie des polymères (US 4 480 125) où la pureté est un critère essentiel.

La présente invention va être plus précisément décrite à l'aide des exemples suivants qui ne doivent pas être considérés comme limitatifs de l'invention.

Les abréviations utilisées dans l'ensemble des exemples signifient :
- TT = taux de transformation = % d'acide itaconique transformé,
- RT = rendement de l'anhydride formé sur l'acide transformé.

EXEMPLE 1 selon l'invention :

a) Description de l'argile :

Le catalyseur employé est une argile de type Montmorillonite à propriétés acides (Réf. : KSF de Sud-Chemie (Munich), RFA).

b) Traitement d'activation de l'argile : KSF - HCl - MeOH

Dans un bécher en verre de 1 litre, muni d'une agitation magnétique, on charge successivement 800 ml d'eau, puis de l'acide chlorhydrique concentré jusqu'à obtention d'un pH = 2,5, puis 5 g d'argile KSF. Si le pH varie après l'addition de l'argile, on réajuste le pH à 2,5.

La suspension est agitée à 25°C pendant 1 heure. L'argile est récupérée par filtration sur verre fritté et lavée avec de l'eau permutée et remise en suspension dans 400 ml de MeOH. Après agitation à 25°C pendant 1 h 30, l'argile est isolée par filtration sur verre fritté, lavée avec du methanol et séchée 16 heures à 40°C sous 100 mm Hg.

c) Engagement du catalyseur KSF - HCl - MeOH dans la réaction d'anhydridation

Dans un réacteur tricol en verre de 100 ml, muni d'une agitation centrale, d'une colonne vigreux (équipée d'un analyseur en tête avec passage de l'azéotrope toluène eau sur une colonne de tamis moléculaire 3A et recyclage du toluène par un siphon), d'une ampoule de coulée de 20 ml, d'une arrivée de gaz et d'un système de chauffage, on charge sous argon 2 g d'acide itaconique, 0,5 g du catalyseur préparé selon 1b) et 50 à 55 ml de toluène. On chauffe à reflux pendant 3 heures.

Après réaction, on laisse refroidir à température ambiante. Le catalyseur est récupéré par filtration sur verre fritté et lavé avec 4 x 30 ml d'acétone (récupération éventuelle de l'acide itaconique adsorbé). Les filtrats sont réunis et les solvants évaporés sous pression réduite (évaporateur rotatif équipé d'une pompe à huile) à 40°C.

L'acide itaconique ainsi que les anhydrides itaconique et citraconique sont dosés par RMN -$^1$H (360 MHz). Une technique d'intégration particulière permet l'obtention des différents titres à plus ou moins 0,25 %. Le taux de transformation de l'acide itaconique est de 93 %, et les sélectivités respectives en anhydride itaconique et citraconique de 94 % et 5 %.

EXEMPLE 2 comparatif :

- Préparation d'une argile échangée Al$^{3+}$

On introduit progressivement 5 g de TONSIL 13 (montmorillonite alcaline (Ca$^{2+}$, Na$^+$) commercialisée par Süd-Chemie dans une solution aqueuse de AlCl$_3$ 0,24 M avec une bonne agitation et à 25°C. Ces conditions sont maintenues pendant 30 minutes. Après centrifugation (5200 tours par minute pendant 8 minutes) le solide est lavé deux fois avec 80 ml d'eau permutée. Chaque lavage étant suivi d'une centrifugation. Le TONSIL 13-Al$^{3+}$ obtenu est séché à 80°C pendant 17 heures.

- Utilisation du TONSIL 13-Al$^{3+}$ dans la réaction d'anhydridation

On procéde comme à l'exemple 1, mais en employant l'argile échangée Al$^{3+}$ comme catalyseur. La conversion de l'acide itaconique est de 62 % et les sélectivités en anhydride itaconique de 91 % et en anhydride citraconique de 7 %.

EXEMPLE 3 :

On procéde comme à l'exemple 1 en employant la même argile traitée HCl aqueux (pH = 2,5) uniquement KSF - HCl. La conversion de l'acide itaconique est de 76 % et les sélectivités en anhydride itaconique de 92 % et en anhydride citraconique de 3 %.

EXEMPLE 4 comparatif :

On procède comme à l'exemple 1 en employant la même argile (KSF) non traitée. La conversion de l'acide itaconique est de 9 % et les sélectivités en anhydride itaconique de 67 % et en anhydride citraconique de 43 %.

EXEMPLES 5 à 8 :

Ces exemples décrivent l'utilisation de divers acides servant à acidifier l'argile et de divers solvants organiques servant à disperser l'argile après acidification.

Ils sont réalisés dans les conditions de l'exemple 1 mais au cours de l'étape c) on introduit 1 g de catalyseur à la place des 0,5 g de l'exemple 1.

| N° ex | ARGILE DE TYPE MONTMORILLONITE (REF D'ORIGINE) | ACIDE | SOLVANT | TT (%) ACIDE ITACONIQUE | RT (%) ANHYDRIDE ITACONIQUE | RT (%) ANHYDRIDE CITRACONIQUE |
|---|---|---|---|---|---|---|
| 5 | KSF | HCL | CH$_3$OH | 96 | 95 | 2 |
| 6 | KSF | F$_3$CSO$_3$H | CH$_3$OH | 96 | 90 | 2 |
| 7 | K10 | HNO$_3$ | (CH$_3$)$_2$ C=O | 94 | 90 | 3 |
| 8 | K10 | H$_2$SO$_4$ | (CH$_3$)$_2$ C=O | 95 | 90 | 4 |

EXEMPLES 9 à 11

Ces exemples décrivent l'utilisation de différents solvants organiques permettant la mise en contact de l'acide itaconique et de l'argile KSF-HCl-Methanol préparée selon l'exemple 1.

| N° Ex | T °C | t h | SOLVANT | TT ACIDE ITACONIQUE % | R T | |
|---|---|---|---|---|---|---|
| | | | | | ANHYDRIDE ITACONIQUE | ANHYDRIDE CITRACONIQUE |
| 9 | 111 | 3 | Toluène | 98 | 96 | 1 |
| 10 | 138 | 1.5 | Xylènes | 97 | 92 | 2 |
| 11 | 138 | 0.7 | Xylènes | 96 | 97 | 1 |

5

## EXEMPLES 12 à 14

Ces exemples permettent de décrire l'utilisation d'argiles différentes de la KSF ou de la K10 utilisées dans les exemples précédents, mais traitées une ou plusieurs fois avec des acides différents et des alcools ou cétones différents (voir tableau ci-après).

### EXEMPLES 12 à 14

| Ex. | Type d'argile | Nom commercial | Acide | Alcool cétone | TT acide itaconique | anhydride itaconique | RT anhydride citraconique |
|---|---|---|---|---|---|---|---|
| 12 | Montmorillonite | VOLCLAY | $H_3PO_4$ | $i.C_3H_7OH$ | 33 | 68 | 14 |
| 13 | Montmorillonite | TONSIL OPTIMUM FF | HCl | $CH_3OH$ | 97 | 93 | 2 |
| 14 | Montmorillonite | COPISIL D4 | HCl | $CH_3OH$ | 14 | 46 | 18 |

EXEMPLES 15 à 23 :

Ces exemples permettent d'illustrer l'anhydridation d'acides différents de l'acide itaconique.
On met en oeuvre dans chaque exemple :
- 16,7 mmoles de substrat,
- 60 ml de solvant dont la nature est indiqué dans chaque tableau,
- 0,5 g à 2 g de catalyseur.
Le catalyseur est préparé selon l'exemple 1 (a à c).
Les températures et durées de réaction sont indiquées dans les tableaux suivants :

Tableau 1


Résultats d'anhydridation dans du toluène


température    110°C

temps de réaction : 3 heures

masse de catalyseur : 0,5 g

| Ex | Catalyseur | Substrat | TT(%) | Produit obtenu | RT(%) |
|----|-----------|----------|-------|---------------|-------|
| 15 | KSF-HCl-MeOH | | 96 | | 95 |
| 16 | KSF-HCl-MeOH | | 66 | | 99 |

7

Tableau 2

<u>Résultats d'anhydridation dans du xylène</u>

température   140°C

temps de réaction : 0,8 à 1 heure

masse de catalyseur : 0,5 g

| Ex | Catalyseur | Substrat | TT(%) | Produit obtenu | RT(%) |
|---|---|---|---|---|---|
| 17 | KSF-HCl-MeOH | | 96 | | 97 |
| 18 | TONSIL OPTIMUM FF | | 95 | | 92 |
| 19 | KSF-HCl-MeOH | | 28 | | 86 |
| 20 | KSF-HCl-MeOH | CIS | 73 | | 100 |
| 21 | KSF-HCl-MeOH | | 100 | | 100 |

Tableau 3

Résultats d'anhydridation dans du mésitylène(1,3,5-triméthylbenzène)

température 165°C

| Ex | Catalyseur | Masse catalyseur (g) | Substrat | TT (%) | Durée (h) | Produit | RT (%) |
|----|-----------|------|----------|--------|-----------|---------|--------|
| 22 | KSF-HCl-MeOH | 1 | | 87 | 1 | | 97 |
| 23 | KSF-HCl-MeOH | 2 | | 36 | 6 | | 64 |

**Revendications**

1. Procédé de préparation d'anhydrides d'acide caractérisé en ce que l'on met en présence un acide devant subir l'anhydridation avec une argile ayant subi un ou plusieurs traitements acides.

2. Procédé selon la revendication 1 caractérisé en ce que les acides devant subir l'anhydridation sont choisis parmi les acides polycarboxyliques, aliphatiques, cycloaliphatiques ou aromatiques.

3. Procédé selon la revendication 2 caractérisé en ce que les acides aliphatiques sont des acides dicarboxyliques saturés sur la chaîne principale

4. Procédé selon la revendication 3 caractérisé en ce que les acides aliphatiques sont choisis parmi l'acide itaconique et l'acide succinique.

5. Procédé selon la revendication 2 caractérisé en ce que les acides cycloaliphatiques sont des acides dicarboxyliques saturés.

6. Procédé selon la revendication 2 caractérisé en ce que les acides aromatiques sont des acides dicarboxyliques.

7. Procédé selon la revendication 1 caractérisé en ce que les traitements acides sont réalisés par des acides choisis parmi l'acide chlorhydrique, l'acide sulfurique, l'acide nitrique, l'acide perchlorique, l'acide phosphorique ou l'acide trifluorométhanesulfonique.

8. Procédé selon la revendication 1 caractérisé en ce que l'on effectue le ou les traitements acides avec une quantité d'acide exprimée en milliéquivalents correspondant au moins à la capacité d'échange de l'argile.

9. Procédé selon la revendication 1 caractérisé en ce qu'on effectue après acidification un traitement par un alcool choisi parmi le méthanol, l'isopropanol ou par une cétone telle que l'acétone.

10. Procédé selon la revendication 1 caractérisé en ce que l'on met en contact l'acide devant subir l'anhydridation et l'argile en présence d'un solvant choisi parmi les solvants organiques aromatiques éventuellement halogénés et les solvants aliphatiques chlorés.

11. Procédé selon la revendication 5 caractérisé en ce que la quantité de solvant utilisée est telle que le rapport pondéral du solvant à l'acide devant subir l'anhydridation est compris entre 20 et 150.

12. Procédé selon la revendication 1 caractérisé en ce que la température de réaction est comprise entre 80 et 200°C.

**Claims**

1. Process for the preparation of acid anhydrides, characterised in that an acid which is to be converted to an anhydride is brought into contact with a clay which has undergone one or more acid treatments.

2. Process according to Claim 1, characterised in that the acids which are to be converted to anhydrides are selected from aliphatic, cycloaliphatic and aromatic polycarboxylic acids.

3. Process according to Claim 2, characterised in that the aliphatic acids are dicarboxylic acids saturated on the main chain.

4. Process according to Claim 3, characterised in that the aliphatic acids are selected from itaconic acid and succinic acid.

5. Process according to Claim 2, characterised in that the cycloaliphatic acids are saturated dicarboxylic acids.

6. Process according to Claim 2, characterised in that the aromatic acids are dicarboxylic acids.

7. Process according to Claim 1, characterised in that the acid treatments are carried out with acids selected from hydrochloric acid, sulphuric acid, nitric acid, perchloric acid, phosphoric acid and trifluoromethanesulphonic acid.

8. Process according to Claim 1, characterised in that the acid treatment or treatments are carried out with an amount of acid, expressed in milliequivalents, which at least corresponds to the exchange capacity of the clay.

9. Process according to Claim 1, characterised in that, after acidification, a treatment is carried out with an alcohol selected from methanol and isopropanol or with a ketone such as acetone.

10. Process according to Claim 1, characterised in that the acid which is to be converted to the anhydride is brought into contact with the clay in the presence of a solvent selected from optionally halogenated aromatic organic solvents and chlorinated aliphatic solvents.

11. Process according to Claim 5, characterised in that the amount of solvent used is such that the weight ratio of the solvent to the acid which is to be converted to the anhydride is between 20 and 150.

12. Process according to Claim 1, characterised in that the reaction temperature is between 80 and 200°C.

**Patentansprüche**

1. Verfahren zur Herstellung von Säureanhydriden, dadurch gekennzeichnet, daß eine Säure, die zu einem Anhydrid umgesetzt werden soll, mit einem Ton in Kontakt gebracht wird, der eine oder mehrere Säurebehandlungen erfahren hat.

2. Verfahren nach Anspruch 1, dadurch gekennzeichnet, daß die Säuren, die zu einem Anhydrid umgesetzt werden sollen, unter den aromatischen, cycloaliphatischen oder aliphatischen Polycarbonsäuren ausgewählt werden.

3. Verfahren nach Anspruch 2, dadurch gekennzeichnet, daß die aliphatischen Säuren Dicarbonsäuren sind, deren Hauptkette gesättigt ist.

4. Verfahren nach Anspruch 3, dadurch gekennzeichnet, daß die aliphatischen Säuren unter der Itaconsäure und der Bernsteinsäure ausgewählt werden.

5. Verfahren nach Anspruch 2, dadurch gekennzeichnet, daß die cycloaliphatischen Säuren gesättigte Dicarbonsäuren sind.

6. Verfahren nach Anspruch 2, dadurch gekennzeichnet, daß die aromatischen Säuren Dicarbonsäuren sind.

7. Verfahren nach Anspruch 1, dadurch gekennzeichnet, daß die Säurebehandlungen mit den Säuren durchgeführt werden, die unter Chlorwasserstoffsäure, Schwefelsäure, Salpetersäure, Perchlorsäure, Phosphorsäure oder Trifluormethansulfonsäure ausgewählt werden.

8. Verfahren nach Anspruch 1, dadurch gekennzeichnet, daß die Säurebehandlung oder die Säurebehandlungen mit einer in Milliequivalenten ausgedrückten Säuremenge durchgeführt werden, die mindestens der Austauschkapazität des Tones entspricht.

9. Verfahren nach Anspruch 1, dadurch gekennzeichnet, daß nach der Säurebehandlung eine Behandlung mit einem Alkohol, der unter Methanol, Isopropanol ausgesucht wird, oder mit einem Keton, wie Aceton, durchgeführt wird.

10. Verfahren nach Anspruch 1, dadurch gekennzeichnet, daß die Säure, die zu einem Anhydrid umgesetzt werden soll, mit einem Ton in Gegenwart eines Lösungsmittels in Kontakt gebracht wird, das unter den eventuell halogenierten aromatischen organischen Lösungsmitteln und den chlorierten aliphatischen Lösungsmitteln ausgesucht wird.

11. Verfahren nach Anspruch 5, dadurch gekennzeichnet, daß die eingesetzte Säuremenge derart ist, daß das Gewichtsverhältnis des Lösungsmittels zur Säure, die zu einem Anhydrid umgesetzt werden soll, zwischen 20 und 150 liegt.

12. Verfahren nach Anspruch 1, dadurch gekennzeichnet, daß die Reaktionstemperatur zwischen 80 und 200°C liegt.